# FASCICULE DE BREVET EUROPEEN

(11) **EP 1 607 068 B1**
(45) Date de publication et mention de la délivrance du brevet: **22.04.2009**
(21) Numéro de dépôt: 05356104.9
(22) Date de dépôt: 14.06.2005
(51) Int. Cl.: A61F 2/40

(54) **Jeu de composants huméraux pour prothèse totale d'épaule**
Satz von Oberarmteilen für Schultertotalprothese
Set of humeral components for total shoulder prothesis

(30) Priorité: 15.06.2004 FR 0406470
(43) Date de publication de la demande: 21.12.2005
(73) Titulaire: TORNIER, 38330 Saint-Ismier (FR)
(72) Inventeur: Tornier, Alain, 38330 Saint Ismier (FR); Sirveaux, François, 54600 Villers les Nancy (FR); Walsch, Gilles, 69003 Lyon (FR); Mole, Daniel, 54000 Nancy (FR); Levigne, Christophe, 69300 Caluire (FR); Boileau, Pascal, 06200 Nice (FR); Favard, Luc, 37270 Montlouis (FR)
(74) Mandataire: Myon, Gérard Jean-Pierre

(56) Documents cités:
- EP-A- 0 299 889
- EP-A- 1 062 923
- WO-A-93/09733
- WO-A-03/005933
- FR-A- 2 699 400
- FR-A- 2 836 039
- US-A- 3 978 528
- US-A- 4 206 517
- US-A- 5 910 171

## Description

L'invention a trait à un jeu de composants huméraux pour une prothèse totale d'épaule, ainsi qu'à une prothèse .

Dans le domaine des prothèses totales d'épaule, il est connu, par exemple de US -A-3,978,528, de EP-A-0 299 889 ou de FR-A-2 836 039, de constituer une prothèse dans laquelle une surface articulaire convexe est solidaire de la glène, alors qu'une surface articulaire concave est solidaire de l'humérus, la coopération de ces surfaces permettant de recréer une articulation au niveau de l'épaule. Avec ces prothèses connues, il peut arriver, lors du mouvement d'adduction, qu'une portion de la partie métaphysaire du composant huméral vienne heurter le pilier de l'omoplate, ce qui limite ce mouvement et peut s'avérer douloureux, voire même induire un descellement de la prothèse.

C'est à ces inconvénients qu'entend plus particulièrement remédier l'invention en proposant un jeu de composants huméraux qui permet au chirurgien d'optimiser le positionnement relatif des composants prothétiques, en fonction de l'anatomie du patient.

Dans cet esprit, l'invention concerne un jeu de composants huméraux pour prothèse totale d'épaule dans lequel chaque composant est formé d'une queue d'ancrage et d'une partie métaphysaire qui définit une surface concave d'articulation globalement en tronçon de sphère. Ce jeu de composants est caractérisé en ce que le décalage entre l'axe central de la partie métaphysaire et l'axe de symétrie de la surface concave d'articulation des différents composants est variable d'un composant à l'autre, tout en étant fixe pour un composant donné.

Grâce à l'invention, la surface articulaire concave des différents composants huméraux peut être positionnée, par rapport à la surface externe de la partie méthaphysaire, d'une façon telle que les interférences de la partie métaphysaire avec le pilier de l'omoplate sont minimisées, voire supprimées.

Selon des aspects avantageux mais non obligatoires, un jeu de composants huméraux peut incorporer une ou plusieurs des caractéristiques suivantes, prises dans toutes combinaisons techniquement admissibles :
- Pour chaque composant, l'axe de symétrie de la surface articulaire et l'axe central de la partie métaphysaire sont sensiblement parallèles.
- Pour l'un au moins de ces composants, l'axe de symétrie de la surface articulaire est disposé, par rapport à l'axe central de la partie métaphysaire, à l'opposé d'un axe médian de la queue d'ancrage. Dans ce cas, la surface d'articulation de l'un au moins de ces composants s'étend avantageusement jusqu'au voisinage immédiat du bord de la partie métaphysaire, voire même est coupée par ce bord, à l'opposé de la partie du bord située globalement dans le prolongement de la queue d'ancrage.
- Les différents composants ont sensiblement la même forme, à l'exception de la position de la surface articulaire dans la partie métaphysaire.
- L'angle d'inclinaison de l'axe de symétrie de la surface articulaire par rapport à l'axe médian de la tige a sensiblement la même valeur pour tous les composants.
- Le décalage précité peut être nul pour l'un des composants huméraux, les axes étant alors confondus.

L'invention concerne également une prothèse totale d'épaule qui comprend un composant huméral sélectionné dans un jeu de composants tel que précédemment décrit et dans lequel le décalage entre l'axe de symétrie de sa surface concave d'articulation et l'axe central de sa partie métaphysaire est non nul. Une telle prothèse est plus facilement adaptable à la morphologie du patient.

Selon un aspect avantageux de l'invention, une telle prothèse comprend en outre un composant glénoïdien qui forme une surface d'articulation convexe centrée sur un axe de symétrie qui n'est pas perpendiculaire à une face arrière de ce composant destiné à venir en appui contre la glène. Cet aspect de l'invention permet de « rattraper » ou de « compenser » un défaut de parallélisme entre la surface réséquée de la glène contre laquelle vient en appui le composant glénoïdien et l'axe de la colonne vertébrale du patient.

Une méthode de pose d'une prothèse totale d'épaule comprend des étapes consistant à :
- préparer les os en vue de la mise en place d'un composant glénoïdien et d'un composant huméral ;
- installer sur la glène un composant glénoïdien définissant une surface articulaire convexe ;
- sélectionner, dans un jeu de composants huméraux, un composant huméral pourvu d'une surface articulaire concave apte à coopérer avec une surface articulaire convexe du composant glénoïdien, ce composant n'interférant pas sensiblement ou interférant relativement peu avec le pilier de l'omoplate lors d'un mouvement d'adduction et
- installer le composant huméral sur l'humérus.

La méthode peut être mise en oeuvre par un chirurgien installant une prothèse totale d'épaule, la sélection du composant huméral le plus adapté pouvant être effectuée au cours de l'opération chirurgicale ou, de façon anticipée au cours du bilan pré-opératoire.

L'invention sera mieux comprise et d'autres avantages de celle-ci apparaîtront plus clairement à la lumière de la description qui va suivre d'un jeu de composants huméraux conforme à son principe et de la pose d'une prothèse totale d'épaule au moyen de ce jeu de composants, donnée uniquement à titre d'exemple et faite en référence aux dessins annexés dans lesquels :
- la figure 1 est une représentation schématique de principe d'une prothèse totale d'épaule installée sur un patient et comprenant un composant huméral représenté en coupe, issu d'un jeu de composants conforme à l'invention ;
- la figure 1A est une vue du détail A à la figure 1 en configuration d'interférence entre le composant huméral et le pilier de l'omoplate ;
- la figure 2 est une vue analogue à la figure 1, alors que le composant huméral est différent de celui utilisé dans la prothèse de la figure 1 ;
- la figure 3 est une représentation schématique d'un jeu conforme à l'invention de composants huméraux représentés en coupe, et
- la figure 4 est une vue analogue à la figure 1A pour une prothèse dont le composant huméral est identique à celui de la figure 2, le composant glénoïdien étant différent.

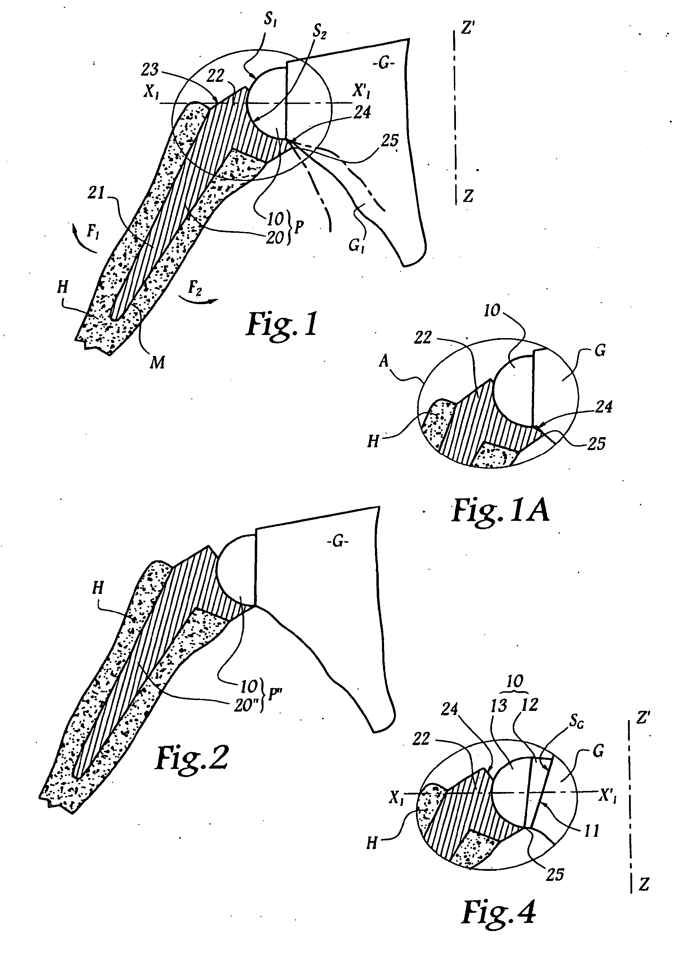

La prothèse P représentée à la figure 1 comprend un composant glénoïdien 10 qui est fixé à la glène G par tout moyen approprié, par exemple conformément à l'enseignement technique de FR-A-2 836 039, et qui définit une surface articulaire convexe S₁ sensiblement en forme de demi-sphère.

La prothèse P comprend également un composant huméral 20 formé d'une queue d'ancrage 21 et d'une partie métaphysaire 22. La queue 21 est globalement rectiligne et apte à être introduite dans le canal médullaire M de l'humérus H, alors que la partie métaphysaire 22 fait saillie au-delà de ce canal et définit une surface articulaire concave S₂ en forme de tronçon de sphère.

Sur les figures 1, 1A et 2, la glène 4 et le composant 10 sont représentés en vue de côté, alors que l'humérus H et le composant 20 sont représentés en coupe longitudinale.

Les surfaces S₁ et S₂ sont adaptées pour constituer une articulation glissante et ont des rayons sensiblement égaux.

La partie métaphysaire 22 est de forme externe globalement cylindrique à base circulaire et l'on note 23 sa surface radiale externe. On note par ailleurs 24 sa surface d'extrémité qui est globalement plane et dans laquelle est creusée la surface concave S₂. On note enfin 25 le bord périphérique externe qui relie les surfaces 23 et 24 et qui est circulaire comme la surface 23.

On définit comme axe central X₂₂ de la partie métaphysaire 22 un axe perpendiculaire à la surface 24 et passant par le centre imaginaire de cette surface. En pratique, l'axe X₂₂ est un axe de symétrie du bord 25.

Dans certaines configurations, la surface 24 peut être supprimée. Le bord 25 relie alors directement les surfaces S₂ et 23. Dans ce cas, l'axe X₂₂ est défini comme l'axe de symétrie du bord 25.

L'humérus H est supposé subir des mouvements d'abduction représentés par la flèche F₁ et des mouvements d'adduction représentés par la flèche F₂ à la figure 1.

En fin de course d'adduction, les parties de la surface 24 et du bord 25 les plus éloignées de la tige 21 sont susceptibles de heurter le pilier de l'omoplate G₁, c'est-à-dire la partie de la glène G située au voisinage du composant 10 en dessous de celui-ci lorsque le patient se tient debout. Dans cette configuration d'interférence représentée à la figure 1A, le patient ressent une gêne, ce qui est préjudiciable à la réussite de l'opération.

On comprend que cette configuration d'interférence n'est pas systématique dans la mesure où le pilier de l'omoplate G₁ peut avoir différentes formes, comme représenté, en traits mixtes uniquement, à la figure 1.

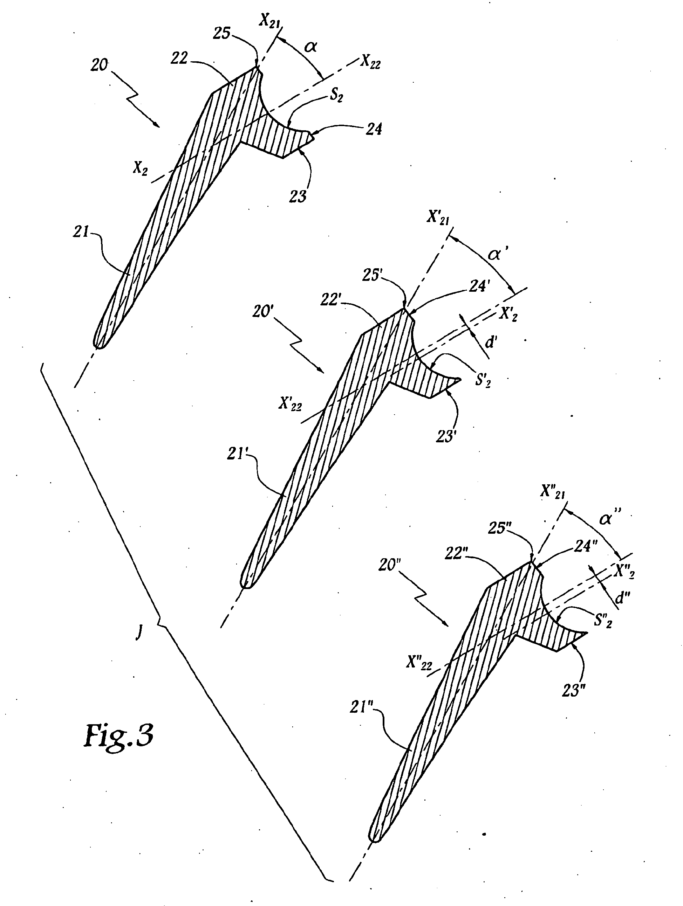

Conformément à l'invention, et ainsi qu'il ressort plus particulièrement de la figure 3, un jeu J de composants huméraux est prévu, dans lequel plusieurs composants 20, 20' et 20" sont préparés avec des géométries différentes.

Comme il ressort de la figure 3, les différents composants 20, 20' et 20'' du jeu J ont globalement la même forme extérieure, leurs queues respectives 21, 21' et 21" ayant sensiblement la même forme extérieure, de même que leurs parties métaphysaires 22, 22' et 22" . En particulier, les surfaces radiales externes 23, 23' et 23" de ces différents parties métaphysaires ainsi que leurs bords 25, 25' et 25" ont sensiblement la même géométrie.

On note respectivement X₂₁, X'₂₁ et X''₂₁ les axes médians des queues d'ancrage 21, 21' et 21" .

On définit par ailleurs comme axe X₂ l'axe de symétrie de la surface S₂.

Pour le composant 20, les axes X₂ et X₂₂ sont confondus.

On définit comme précédemment l'axe X'₂₂ comme l'axe central de la partie métaphysaire 22' pour le composant 20' et l'axe X'₂ comme l'axe de symétrie de la surface d'articulation concave S'₂ du composant 20'. Les axes X'₂ et X'₂₂ sont parallèles entre eux et décalés d'une distance d' non nulle. De la même façon, on définit les axes X"₂₂ et X"₂ comme étant respectivement l'axe central de la partie métaphysaire 22" et l'axe de symétrie de la surface S"₂, ces axes étant parallèles et décalés d'une distance d" supérieure à la distance d'.

On note α l'angle d'inclinaison entre les axes X₂₁ et X₂₂, cet angle étant également l'angle d'inclinaison entre les axes X₂ et X₂₁ puisque les axes X₂ et X₂₂ sont parallèles. L'angle α' entre les axes X'₂₁ et X'₂₂ et l'angle α" entre les axes X"₂₁ et X"₂₂ ont la même valeur que l'angle α.

Cependant, une telle égalité des angles α, α' et α" n'est pas obligatoire dans la mesure où les axes X₂ et X₂₂, X'₂ et X'₂₂, X''₂ et X"₂₂ ne sont pas nécessairement parallèles.

Les différents composants 20, 20' et 20" du jeu J se distinguent donc les uns des autres par le fait que leur surface d'articulation concave S₂, S'₂, S"₂ est plus ou moins décalée par rapport à l'axe central de leur surface d'extrémité 24, 24' ou 24" . Sauf pour le composant 20, les axes X'₂ ou équivalents des surfaces articulaires concaves sont plus proches que les axes X'₂₂ et X''₂₂ de la partie du bord 25 la plus éloignée de l'axe X'₂₁ ou équivalent. En d'autres termes, les surfaces S'₂ et S"₂ sont décalées vers le bas à la figure 3 par rapport à la position médiane qu'occupe la surface S₂ vis-à-vis des surfaces 23 et 24 du composant 20.

La surface concave S"₂ du composant 20" se prolonge jusqu'au voisinage immédiat du bord 25" dans sa partie la plus éloignée de la tige 21", ce qui induit que la surface 24" est de largeur nulle pratiquement dans cette zone. Les risques d'interférences avec le pilier de l'omoplate G₁ sont donc particulièrement limités dans ce cas.

Ainsi, et comme représenté à la figure 2, une prothèse P" équipée du composant 20" a moins de chance d'interférer avec le pilier G₁ de l'omoplate, même si celui-ci a la même géométrie que celui représenté en trait plein à la figure 1.

Lors de la pose d'une prothèse d'épaule, le chirurgien effectue les coupes osseuses et prépositionne le composant glénoïdien ou un composant fantôme. Il peut alors sélectionner dans le jeu J le composant huméral le plus adapté, éventuellement après essai avec des composants fantômes, pour minimiser les risques d'interférence. En variante, le chirurgien peut pré-sélectionner le composant huméral à utiliser lors du bilan préopératoire.

L'invention permet donc au chirurgien, par une sélection raisonnée du composant huméral 20, 20', 20" ou équivalent dans le jeu J de composants, d'adapter la position relative des composants glénoïdien et huméral lorsque leurs surfaces d'articulation respectives coopèrent, tout en évitant ou en limitant dans une très large mesure les interférences entre un bord 25 ou équivalent du composant huméral et le pilier de l'omoplate. L'invention permet donc de rendre plus vertical ou de « verticaliser » l'humérus en fin de course d'adduction.

Dans le jeu J, les composants huméraux ont sensiblement la même géométrie extérieure, à l'exception du positionnement de leur surface articulaire concave. Il est bien entendu que les différents jeux J peuvent être prévus dans différentes tailles pour s'adapter aux morphologies des patients à traiter ou qu'un jeu J de composants huméraux peut incorporer des composants de hauteurs différentes. En outre, le nombre de composants d'un même jeu n'est pas limité à trois et peut prendre toute valeur supérieure à deux, en fonction de la précision recherchée pour l'ajustement du décalage d', d", etc...

Selon une variante non représentée de l'invention, le décalage entre les axes X₂ et X₂₂ peut être encore plus important que celui référencé d" à la figure 3, auquel cas la surface S₂ est coupée par le bord 25. Une telle variante permet de décaler encore plus la surface S₂ vers le bas, étant entendu que le centre de rotation se situe sur la surface S₂.

Comme représenté à la figure 4, la surface fraisée S_{G} de la glène n'est pas toujours parallèle à un axe vertical Z-Z' passant par le centre de la colonne vertébrale du patient qui se tient debout. Or, pour une bonne coopération des surfaces S₁ et S₁, il est préférable que l'axe de symétrie X₁-X'₁ de la surface hémisphérique S₁ soit sensiblement perpendiculaire à l'axe Z-Z'. C'est pourquoi, dans le cas où la surface S_{G} est inclinée comme représenté à la figure 4, on utilise un composant glénoïdien 10 dont la face arrière 11 n'est pas perpendiculaire à l'axe X₁-X'₁, ce qui permet de positionner le composant 10 de telle sorte que cet axe X₁-X'₁ est sensiblement perpendiculaire à l'axe Z-Z'.

Le composant 10 peut être formé d'une embase 12 et d'une coiffe 13, définissant la surface S₁ et montée sur l'embase 12. La face arrière 11 de l'embase 12 est alors avantageusement non parallèle à sa face avant sur laquelle est montée la coiffe 13.

De façon avantageuse, un composant 10 tel que représenté à la figure 4 peut être sélectionné dans un jeu de composants présentant des différences d'orientation variables entre leurs faces arrières respectives et l'axe des surfaces S₁ qu'ils définissent.

L'invention a été représentée avec des composants huméraux monoblocs. En pratique et selon un aspect non représenté de l'invention, les parties métaphysaires de ces composants sont le plus souvent équipées de coupelles en matière plastique définissant les surfaces S₂ de ces composants.

Selon une variante non représentée de l'invention, l'axe de symétrie de la surface S₁ peut être décalé, vers le bas lorsque le patient se tient debout, par rapport à l'axe de symétrie de l'embase sur laquelle est montée la coiffe définissant cette surface.

Ceci peut être combiné avec la non perpendicularité de l'axe X₁-X'₁ et de la face arrière de la prothèse mentionnée ci-dessus en référence au mode de réalisation de la figure 4.

## Revendications

1. Jeu de composants huméraux pour prothèse totale d'épaule dans lequel chaque composant est formé d'une queue d'ancrage et d'une partie métaphysaire qui définit une surface .concave d'articulation globalement en tronçon de sphère, **caractérisé en ce que** le décapage (d', d") entre l'axe de symétrie (X₂, X'₂, X"₂) de ladite surface (S₂, S'₂, S"₂) et l'axe central (X₂₂, X'₂₂, X"₂₂) de ladite partie métaphysaire (22, 22', 22") desdits composants (20, 20', 20") est variable d'un composant à l'autre, tout en étant fixe pour un composant donné.

2. Jeu de composants selon la revendication 1, **caractérisé en ce que**, pour chaque composant (20 : 20', 20"), lesdits axes (X₂, X'₂, X"₂, X₂₂, X'₂₂, X"₂₂) sont sensiblement' parallèles.

3. Jeu de composants selon l'une des revendications précédentes, **caractérisé en ce que**, pour l'un au moins (20', 20") desdits composants, ledit axe de symétrie (X'₂, X"₂) est situé, par rapport audit axe central (X'₂₂, X"₂₂), à l'opposé d'un axe médian (X'₂₁, X"₂₁) de ladite queue (21', 21").

4. Jeu de composants selon la revendication 3, **caractérisé en ce que** la surface d'articulation (S"₂) de l'un au moins desdits composants s'étend jusqu'au voisinage immédiat du bord (25") de ladite partie métaphysaire (22"), à l'opposé de la partie dudit bord située globalement dans le prolongement de ladite queue (21").

5. Jeu de composants selon la revendication 3, **caractérisé en ce que** la surface d'articulation de l'un au moins desdits composants est coupée par ledit bord de ladite partie métaphysaire, à l'opposé de la partie dudit bord située globalement dans le prolongement de ladite queue.

6. Jeu de composants selon l'une des revendications précédentes, **caractérisé en ce que** les différents composants (20, 20', 20") ont sensiblement la même forme, à l'exception de la position de ladite surface articulaire (S₂, S'₂, S"₂) dans ladite partie métaphysaire (22, 22', 22") .

7. Jeu de composants selon l'une des revendications précédentes, **caractérisé en ce que** l'angle d'inclinaison (α, α', α" ) dudit axe de symétrie (X₂, X'₂, X"₂) par rapport à un axe médian (X₂₁, X'₂₁, X"₂₁) de ladite queue (21, 21' , 21") a sensiblement la même valeur pour tous les composants (20, 20', 20").

8. Jeu de composants selon l'une des revendications précédentes, **caractérisé en ce que** pour l'un (20) desdits composants, ledit décalage est nul, lesdits axes (X₂, X₂₂) étant confondus.

9. Prothèse totale d'épaule **caractérisée en ce qu'**elle comprend un composant huméral (20', 20") sélectionné dans un jeu de composants selon l'une des revendications 1-7 et dans lequel le décalage (d', d") entre l'axe de symétrie (X'₂, X"₂) de sa surface concave d'articulation (S'₂, S"₂) et l'axe central (X'₂₂, X"₂₂) de sa partie métaphysaire (22', 22") est non nul.

10. Prothèse selon la revendication 9, **caractérisée en ce qu'**elle comprend en outre un composant glénoïdien (10) formant une surface d'articulation convexe centrée (S₁) sur un axe de symétrie (X₁-X'₁) qui n'est pas perpendiculaire à une face arrière (11) dudit composant destinée à venir en appui contre la glène (G).

## Claims

1. A set of humeral components for a total shoulder prosthesis, in which each component is composed of an anchoring stem and a metaphyseal part which defines a concave joint surface taking the overall form of a portion of a sphere, **characterised in that** the offset (d', d") between the axis of symmetry (X₂, X'₂, X"2) of said surface (S₂, S'₂, S"₂) and the central axis (X₂₂, X'₂₂, X"₂₂) of said metaphyseal part (22, 22', 22") of said components (20, 20', 20") is variable from one component to another while being fixed for a given component.

2. A set of components according to claim 1, **characterised in that**, for each component (20; 20', 20"), said axes (X₂, X'₂, X"₂, X₂₂, X'₂₂, X"₂₂) are substantially parallel.

3. A set of components according to one of the preceding claims, **characterised in that**, for one at least (20', 20") of said components, said axis of symmetry (X'₂, X"₂) is situated, relative to said central axis (X'₂₂, X"₂₂) , on the opposite side from a median axis (X'₂₁, X"₂₁) of said stem (21', 21").

4. A set of components according to claim 3, **characterised in that** the joint surface (S"₂) of one at least of said components extends as far as the immediate vicinity of the edge (25") of said metaphyseal part (22"), on the opposite side from the part of said edge situated overall as a continuation of said stem (21").

5. A set of components according to claim 3, **characterised in that** the joint surface of one at least of said components is intersected by said edge of said metaphyseal part, on the opposite side from the part of said edge situated overall as a continuation of said stem.

6. A set of components according to one of the preceding claims, **characterised in that** the various components (20, 20', 20") have substantially the same shape, with the exception of the position of said joint surface (S₂, S'₂, S"₂) in said metaphyseal part (22, 22', 22").

7. A set of components according to one of the preceding claims, **characterised in that** the angle of inclination (α, α', α") of said axis of symmetry (X₂, X'₂, X"₂) relative to a median axis (X₂₁, X'₂₁, X"₂₁) of said stem (21, 21', 21") has substantially the same value for all the components (20, 20', 20").

8. A set of components according to one of the preceding claims, **characterised in that**, for one (20) of said components, said offset is zero, said axes (X₂, X₂₂) coinciding with one another.

9. A total shoulder prosthesis, **characterised in that** it comprises a humeral component (20', 20") selected from a set of components according to one of claims 1 - 7 and in which the offset (d', d") between the axis of symmetry (X'₂, X"₂) of its concave j oint surface (S'₂, S"₂) and the central axis (X'₂₂, X"₂₂) of its metaphyseal part (22', 22") is not zero.

10. A prosthesis according to claim 9, **characterised in that** it further comprises a glenoid component (10) forming a convex joint surface (S₁) centred on an axis of symmetry (X₁-X'₁) which is not perpendicular to a rear face (11) of said component designed to come to bear against the glenoid (G).

## Patentansprüche

1. Satz von Oberarmkomponenten für eine Schultertotalprothese, bei dem jede Komponente von einem Endteil zur Verankerung und einem Metaphysenteil gebildet ist, der eine konkave Gelenkfläche im Wesentlichen in Form eines Kugelabschnitts definiert, **dadurch gekennzeichnet, dass** der Versatz (d', d") zwischen der Symmetrieachse (X₂, X'₂, X"₂) der Fläche (S₂, S'₂, S"₂) und der Zentralachse (X₂₂, X'₂₂, X"₂₂) des Metaphysenteils (22, 22', 22") der Komponenten (20, 20', 20") von einer Komponente zur anderen variabel ist, wobei er für eine gegebene Komponente feststehend ist.

2. Satz von Komponenten nach Anspruch 1, **dadurch gekennzeichnet, dass** für jede Komponente (20, 20', 20") die Achsen (X₂, X'₂, X"₂, X₂₂, X'₂₂ X"₂₂) im Wesentlichen parallel sind.

3. Satz von Komponenten nach einem der vorhergehenden Ansprüche, **dadurch gekennzeichnet, dass** für mindestens eine (20', 20") der Komponenten die Symmetrieachse (X'₂, X"₂) in Bezug zur Zentralachse (X'₂₂, X"₂₂) gegenüberliegend einer Mittelachse (X'₂₁, X"₂₁) des Endteils (21', 21") angeordnet ist.

4. Satz von Komponenten nach Anspruch 3, **dadurch gekennzeichnet, dass** sich die Gelenkfläche (S"₂) mindestens einer der Komponenten bis in unmittelbare Nähe des Randes (25") des Metaphysenteils (22") gegenüberliegend dem Teil des Randes erstreckt, der sich im Wesentlichen in der Verlängerung des Endteils (21") befindet.

5. Satz von Komponenten nach Anspruch 3, **dadurch gekennzeichnet, dass** die Gelenkfläche mindestens einer der Komponenten vom Rand des Metaphysenteils gegenüberliegend dem Teil des Randes geschnitten wird, der sich im Wesentlichen in der Verlängerung des Endteils befindet.

6. Satz von Komponenten nach einem der vorhergehenden Ansprüche, **dadurch gekennzeichnet, dass** die verschiedenen Komponenten (20, 20', 20") im Wesentlichen dieselbe Form haben, mit Ausnahme der Position der Gelenkfläche (S₂, S'₂, S"₂) in dem Metaphysenteil (22, 22', 22") .

7. Satz von Komponenten nach einem der vorhergehenden Ansprüche, **dadurch gekennzeichnet, dass** der Neigungswinkel (α, α', α") der Symmetrieachse (X₂, X'₂, X"₂) in Bezug zu einer Mittelachse (X₂₁, X'₂₁, X"₂₁) des Endteils (21, 21', 21") im Wesentlichen denselben Wert für alle Komponenten (20, 20', 20") hat.

8. Satz von Komponenten nach einem der vorhergehenden Ansprüche, **dadurch gekennzeichnet, dass** für eine (20) der Komponenten der Versatz gleich null ist, wobei die Achsen (X₂, X₂₂) zusammenfallen.

9. Schultertotalprothese, **dadurch gekennzeichnet, dass** sie eine Oberarmkomponente (20', 20") umfasst, die aus einem Satz von Komponenten nach einem der Ansprüche 1 bis 7 ausgewählt wird, und bei der der Versatz (d', d") zwischen der Symmetrieachse (X'₂, X"₂) ihrer konkaven Gelenkfläche (S'₂, S"₂) und der Zentralachse (X'₂₂, X"₂₂) ihres Metaphysenteils (22', 22") ungleich null ist.

10. Prothese nach Anspruch 9, **dadurch gekennzeichnet, dass** sie ferner eine Schultergelenkkomponente (10) umfasst, die eine konvexe Gelenkfläche (S₁) bildet, die auf einer Symmetrieachse (X₁ - X'₁) zentriert ist, die nicht senkrecht zu der Rückseite (11) der Komponente ist, die am Schultergelenk (G) zur Anlage gelangen soll.
